# FASCICULE DE BREVET EUROPEEN

(11) **EP 3 986 610 B1**
(45) Date de publication et mention de la délivrance du brevet: **04.03.2026**
(21) Numéro de dépôt: 20786016.4
(22) Date de dépôt: 07.09.2020
(51) Int. Cl.: B01J 20/32, C08F 230/02, B01J 20/28

(54) **PROCÉDÉ DE PRÉPARATION D'UN SUPPORT MONOLITHIQUE SUR LEQUEL DES CATIONS URANYLE SONT IMMOBILISÉS, PROCÉDÉS DE CAPTURE ET DE RÉCUPÉRATION ASSOCIÉS**
VERFAHREN ZUR HERSTELLUNG EINES MONOLITHISCHEN TRÄGERS MIT DARAUF IMMOBILISIERTEN URANYLKATIONEN SOWIE ZUGEHÖRIGE VERFAHREN ZUR ERFASSUNG UND RÜCKGEWINNUNG
METHOD FOR PREPARING A MONOLITHIC SUPPORT ON WHICH URANYL CATIONS ARE IMMOBILISED, AND ASSOCIATED METHODS FOR CAPTURE AND RECOVERY

(30) Priorité: 12.09.2019 FR 1910077
(43) Date de publication de la demande: 27.04.2022
(73) Titulaire: Commissariat à l'Energie Atomique et aux Energies Alternatives, 75015 Paris (FR); Centre National de la Recherche Scientifique, 75794 Paris Cedex 16 (FR); Université Paris-Saclay, 91190 Gif-sur-Yvette (FR)
(72) Inventeur: BRESSON, Carole, 91120 PALAISEAU (FR); GARCIA-CORTES, Marta, 33013 OVIEDO (ES); VIDAUD, Claude, 84420 PIOLENC (FR); TRAN, Thuy, 91370 VERRIERES LE BUISSON (FR)
(74) Mandataire: Santarelli
(86) Numéro de dépôt international: PCT/FR2020/051537
(87) Numéro de publication internationale: WO 2021/048488

(56) Documents cités:
- WO-A1-2019/008278
- US-A1- 2014 178 252
- BASSET ET AL: "Specific capture of uranyl protein targets by metal affinity chromatography", JOURNAL OF CHROMATOGRAPHY A, ELSEVIER, AMSTERDAM, NL, vol. 1185, no. 2, 8 February 2008 (2008-02-08), pages 233 - 240, XP022512258, ISSN: 0021-9673, DOI: 10.1016/J.CHROMA.2008.01.081

## Description

### Domaine technique

La présente invention se rapporte à un procédé de préparation d'un support monolithique sur lequel des cations uranyle sont immobilisés, ce support monolithique étant plus particulièrement synthétisé et ancré *in situ* dans le ou les canaux d'un système analytique miniaturisé.

La présente invention se rapporte également à un procédé de capture de protéines liant sélectivement l'uranium immobilisé sur un tel support monolithique.

L'invention se rapporte enfin à un procédé de récupération de protéines liant sélectivement l'uranium, ce procédé de récupération mettant en œuvre le procédé de capture précédent.

### État de la technique antérieure

Des effets neurotoxiques induits par de faibles concentrations d'uranium sont suspectés chez l'homme. Si la bio-distribution de l'uranium dans l'organisme humain est bien décrite, les mécanismes biochimiques, qui se produisent au niveau cellulaire et moléculaire et qui seraient responsables de ces effets neurotoxiques, restent encore à élucider. L'identification de molécules-cibles, en particulier des protéines liant sélectivement l'uranium, dans un modèle cellulaire neuronal humain, doit permettre de prédire les espèces protéines-uranium qui sont susceptibles de se former et de décrire finement les mécanismes biochimiques associés à la neurotoxicité de l'uranium.

Pour identifier de telles molécules-cibles, la publication de C. Basset et al. ("Specific capture of uranyl protein targets by metal affinity chromatography", Journal of Chromatography A, 2008, 1185, 233-240), référencée **[1]** à la fin de la présente description, a décrit l'exploitation du mode de séparation par chromatographie d'affinité pour ions métalliques immobilisés (en anglais, "Immobilized Metal Affinity Chromatography" et abrégé "IMAC") afin de capturer sélectivement les protéines liant l'uranium sous sa forme uranyle UO₂²⁺.

Dans la suite de la présente description, l'expression "protéines liant sélectivement l'uranium" pourra être utilisée en lieu et place de l'expression "protéines liant sélectivement l'uranium sous sa forme uranyle UO₂²⁺".

Dans la publication **[1],** les expériences de capture sélective, basée sur le mode IMAC, ont été conduites avec un support formé par des microbilles de copolymère de styrène et de divinylbenzène fonctionnalisées par des groupes aminophosphonate (Duolite^{®} C467) par l'intermédiaire desquels les ions uranyle ont été immobilisés. La présence des groupements aminophosphonate s'est révélée satisfaisante pour immobiliser, par complexation, les ions uranyle et conserver des liaisons uranyle libres qui se lient avec les protéines, et notamment les protéines-cibles de l'uranium contenues dans des échantillons de sérum complexe humain.

Comme rapporté dans la publication de A. Dedieu et al. ("Identification of uranyl binding proteins from human kidney-2 cell extracts by immobilized uranyl affinity chromatography and mass spectrometry", Journal of Chromatography A, 2009, 1216, 5365-5376), référencée **[2],** le support décrit dans la publication **[1]** a été utilisé pour la capture et l'identification de protéines liant sélectivement l'uranium contenues dans des extraits de cellules rénales humaines HK-2. Ces protéines ont été capturées en mode discontinu (par lots ou batch, en anglais) puis identifiées par protéomique.

Les publications **[1]** et **[2]** rapportent donc les premiers travaux qui ont été conduits pour immobiliser les ions UO₂²⁺ pour la capture de protéines liant sélectivement l'uranium et leur identification.

Toutefois, la capture ainsi que l'identification des protéines-cibles de l'uranium présentes dans des extraits cellulaires restent un défi, dans la mesure où ces extraits cellulaires ne sont disponibles que dans des quantités très limitées. Par conséquent, les protéines-cibles ne sont présentes dans ces extraits cellulaires qu'en faible abondance. De plus, la méthode de capture en mode discontinu nécessite d'utiliser un volume minimal de 50 µL de microbilles dans un système en suspension, imposant en contrepartie l'engagement de 20 µg à 50 µg d'échantillons protéiques. Une des conséquences directes majeures est la difficulté à réaliser des répliques expérimentales qui sont indispensables pour valider la répétabilité de la méthode de capture, mais aussi de l'identification, de ces protéines-cibles de l'uranium contenues dans ces extraits cellulaires.

Il y a donc un besoin impératif de réduire l'échelle du procédé de capture des protéines-cibles de l'uranium compte tenu de la très faible disponibilité des échantillons biologiques, de la faible abondance de ces protéines-cibles et des limites du mode discontinu.

La miniaturisation d'un support formé par des microbilles présente toutefois un certain nombre de verrous techniques : non seulement le remplissage par des microbilles de canaux de systèmes analytiques miniaturisés est, d'une part, laborieux et, d'autre part, peu reproductible, mais il requiert, en outre, la mise en place de frittés pour le maintien de ces microbilles. Or, les frittés peuvent être à l'origine de la formation de bulles d'air et/ou de phénomènes d'adsorption de solutés au cours des analyses. Tous ces éléments génèrent des problèmes de reproductibilité non négligeables pour les expériences de capture sélective.

On connaît, par ailleurs, le document WO 2019/008278 A1 qui se rapporte à un procédé de fabrication de colonnes de chromatographie comprenant des phases stationnaires monolithiques ainsi que le document US 2014/0178252 A1 qui se rapporte à des dispositifs microfluidiques contenant des monolithes poreux.

Toutefois, aucun de ces deux documents ne décrit que ces colonnes de chromatographie et dispositifs microfluidiques sont dotés de phases stationnaires monolithiques sur lesquelles sont immobilisés des cations UO₂².

Le but de la présente invention est, par conséquent, de pallier les inconvénients de l'art antérieur et de proposer un procédé de préparation d'un support sur lequel des cations UO₂²⁺ sont immobilisés, ce support permettant de capturer en mode continu les protéines liant sélectivement l'uranium qui sont contenues dans un échantillon biologique quel qu'en soit le volume.

Un autre but de la présente invention est de proposer un procédé de capture de protéines liant sélectivement l'uranium, ces protéines étant contenues dans un échantillon biologique, ce procédé présentant une répétabilité et une robustesse améliorées par rapport à celles des procédés de capture décrits dans les publications **[1]** et **[2].**

### Exposé de l'invention

Les buts précédemment énoncés ainsi que d'autres sont atteints, en premier lieu, par un procédé de préparation d'un support sur lequel des cations UO₂²⁺ sont immobilisés, plus particulièrement par un procédé de préparation, dans le volume intérieur d'au moins un canal d'un système analytique miniaturisé, d'un support monolithique sur lequel des cations UO₂²⁺ sont immobilisés.

Selon l'invention, ce procédé comprend les étapes (a) à (e) successives suivantes :
(a) l'activation de la surface interne du ou des canaux ;
(b) l'introduction d'une solution de polymérisation de synthèse d'un support monolithique dans le volume intérieur du ou des canaux, la solution de polymérisation comprenant :
   - un monomère comprenant un groupement phosphate,
   - au moins un agent de réticulation,
   - plusieurs solvants, et
   - un amorceur de polymérisation radicalaire ;
(c) la polymérisation de la solution de polymérisation obtenue à l'étape (b), moyennant quoi on obtient un support monolithique ancré sur les parois du ou des canaux ;
(d) le rinçage du support monolithique obtenu à l'étape (c) ; et
(e) la mise en contact du support monolithique rincé obtenu à l'étape (d) avec une solution comprenant des cations UO₂²⁺, moyennant quoi on obtient le support monolithique sur lequel des cations UO₂²⁺ sont immobilisés.

Le choix de ce monomère comprenant un groupement phosphate, en combinaison avec un agent de réticulation, les solvants et l'amorceur de polymérisation radicalaire, permet de préparer, de manière localisée et *in situ,* dans le volume intérieur du ou des canaux du système analytique miniaturisé, un support monolithique présentant une structure polymérique tridimensionnelle poreuse stable mécaniquement et chimiquement sur laquelle des cations UO₂²⁺ sont immobilisés.

Comme indiqué précédemment, le procédé de préparation d'un support monolithique sur lequel des cations UO₂²⁺ sont immobilisés selon l'invention comprend les étapes (a) à (e) successives.

L'étape (a) d'activation a pour effet de fonctionnaliser la surface interne du ou des canaux de manière à permettre l'ancrage ultérieur du support monolithique sur ses ou leurs parois internes.

Dans le cas où le ou les canaux sont en verre, l'étape (a) d'activation peut être réalisée par silanisation. En effet, la réaction de silanisation permet de modifier les groupements silanol de la surface interne des canaux en verre en groupes vinyle.

On peut, par exemple, envisager d'utiliser du gamma-méthacryloxy-propyltriméthoxysilane (γ-MAPS) comme agent de silanisation.

La solution de polymérisation de synthèse du support monolithique qui est introduite dans le volume intérieur du ou des canaux lors de l'étape (b) du procédé de préparation selon l'invention peut être réalisée par un mélange du monomère comprenant un groupement phosphate, du ou des agents de réticulation, des solvants et de l'amorceur de polymérisation radicalaire.

La solution de polymérisation comprend généralement un seul monomère comprenant un groupement phosphate.

Ce monomère peut notamment être choisi parmi les monomères méthacrylates comprenant un groupement phosphate ou tout autre monomère permettant de lier de façon covalente un groupement phosphaté à la surface d'un support solide monolithique.

Dans une variante du procédé de préparation selon l'invention, la proportion massique du monomère comprenant un groupe phosphate, par rapport à la masse totale de la solution de polymérisation, est comprise entre 4 % en masse et 10 % en masse.

Il est précisé que les expressions "compris(e) entre ... et ..." et "comprend de ... à ..." qui sont utilisées dans la présente demande doivent être comprises comme définissant non seulement les valeurs de l'intervalle, mais également les valeurs des bornes de cet intervalle.

De manière plus particulièrement avantageuse, la proportion massique du monomère comprenant un groupe phosphate, par rapport à la masse totale de la solution de polymérisation, est comprise entre 8,7 % en masse et 9,7 % en masse.

Dans une variante préférentielle du procédé de préparation selon l'invention, la solution de polymérisation mise en œuvre dans l'étape (b) comprend un monomère méthacrylate comprenant un groupement phosphate, ce monomère méthacrylate étant, plus préférentiellement encore, le méthacrylate de polyéthylène glycol phosphate (EGMP).

La solution de polymérisation mise en œuvre dans l'étape (b) du procédé de préparation selon l'invention comprend, par ailleurs, au moins un agent de réticulation.

En d'autres termes, la solution de polymérisation peut ne comprendre qu'un agent de réticulation mais peut tout aussi bien comprendre un mélange de deux, trois, voire plus, agents de réticulation.

Ce ou ces agents de réticulation peuvent notamment être choisis parmi un mélange d'acrylamide (AA) et de bisacrylamide (BAA), le bisacrylamide (BAA), le diméthacrylate d'éthylène et un dérivé de diméthacrylate d'éthylène.

Dans une variante avantageuse, l'agent de réticulation est composé d'un mélange acrylamide/bisacrylamide (AA/BAA), de préférence dans un rapport massique 19/1.

Dans une variante du procédé de préparation selon l'invention, la proportion massique du mélange AA/BAA, de préférence dans un rapport massique 19/1 (AA/BAA), par rapport à la masse totale de la solution de polymérisation, est comprise entre 3 % en masse et 8 % en masse.

De manière plus particulièrement avantageuse, la proportion massique du mélange AA/BAA, de préférence dans un rapport massique 19/1 (AA/BAA), par rapport à la masse totale de la solution de polymérisation, est comprise entre 6,6 % en masse et 7,3 % en masse.

La solution de polymérisation mise en œuvre dans l'étape (b) du procédé de préparation selon l'invention comprend également plusieurs solvants.

Ces solvants peuvent notamment être choisis parmi le dodécanol (DOC), le diméthylsulfoxyde (DMSO) et le diméthylformamide (DMF).

Dans une variante avantageuse du procédé de préparation selon l'invention, la solution de polymérisation mise en œuvre dans l'étape (b) comprend trois solvants, qui sont, préférentiellement, le dodécanol, le diméthylformamide et le diméthylsulfoxyde.

Dans cette variante avantageuse, la proportion massique du dodécanol, par rapport à la masse totale de la solution de polymérisation, est comprise entre 28 % en masse et 59 % en masse et, préférentiellement, entre 52,7 % en masse et 58,3 % en masse ; la proportion massique du diméthylsulfoxyde, par rapport à la masse totale de la solution de polymérisation, est comprise entre 22 % en masse et 53 % en masse et, préférentiellement, entre 22,8 % en masse et 25,2 % en masse ; et la proportion massique du diméthylformamide, par rapport à la masse totale de la solution de polymérisation, est comprise entre 7 % en masse et 9 % en masse.

La solution de polymérisation mise en œuvre dans l'étape (b) du procédé de préparation selon l'invention comprend, en outre, un amorceur de polymérisation radicalaire.

Cet amorceur de polymérisation radicalaire peut notamment être choisi parmi les amorceurs de polymérisation radicalaire qui peuvent être utilisés pour la préparation de supports monolithiques, à savoir l'azobisisobutyronitrile (AIBN), la 2,2-diéthoxyacétophénone (DEA), les *α*-dialkoxyacétophénones, la 2,2-diméthyl-2-hydroxyacétophénone (DARO), les *α*-hydroxyacétophénones, le benzoïne méthyl éther (BME), la 2-méthyl-4'-(méthylthio)-2-morpholino-propiophénone (IRG), les *α-*alkylaminoacétophénones ou encore la 2,2-diméthoxy-2-phénylacétophénone (DMPA).

Dans une variante du procédé de préparation selon l'invention, la proportion massique d'amorceur de polymérisation radicalaire, par rapport à la masse totale de la solution de polymérisation, est comprise entre 0,05 % en masse et 0,2 % en masse.

De manière plus particulièrement avantageuse, la proportion massique d'amorceur de polymérisation radicalaire, par rapport à la masse totale de la solution de polymérisation, est comprise entre 0,14 % en masse et 0,16 % en masse.

Dans une variante préférentielle du procédé de préparation selon l'invention, la solution de polymérisation met en œuvre l'azobisisobutyronitrile (AIBN) comme amorceur de polymérisation radicalaire.

Il est à noter qu'une solution de polymérisation de synthèse d'un support monolithique comprenant du méthacrylate de polyéthylène glycol phosphate, de l'acrylamide, du bisacrylamide, du dodécanol, du diméthylsulfoxyde, du diméthylformamide et de l'azobisisobutyronitrile a été très récemment décrite dans la publication de M. Araya-Farias et al. ("A lab-on-chip for monolith-based preconcentration and electrophoresis separation of phosphopeptides", Analyst, 2017, 142, 485-494), référencée **[3],** pour pré-concentrer des phosphopeptides, qui sont des biomarqueurs potentiels de la maladie d'Alzheimer. À cette fin, des cations Zr⁴⁺ ont été immobilisés à la surface d'un support monolithique phosphaté synthétisé *in situ* et ancré dans le microcanal d'une puce commerciale en forme de croix. Le système analytique miniaturisé intégrant un tel support monolithique a démontré d'excellentes performances en termes de sélectivité et de facteur d'enrichissement des phosphopeptides.

Or, de manière inattendue et surprenante, les Inventeurs ont observé que le support décrit dans la publication **[3]** est tout à fait adapté pour une application autre que celle décrite dans cette publication, non plus pour l'immobilisation des cations Zr⁴⁺ et la capture de certains phosphopeptides, mais pour l'immobilisation des cations UO₂²⁺, la capture des protéines liant sélectivement l'uranium ainsi que leur récupération.

Dans une version particulièrement préférée, la solution de polymérisation qui est introduite dans le volume intérieur du ou des canaux lors de l'étape (b) du procédé de préparation selon l'invention comprend, par rapport à la masse totale de la solution de polymérisation :
- de 4 % en masse à 10 % en masse et, avantageusement, de 8,7 % en masse à 9,7 % en masse de méthacrylate de polyéthylène glycol phosphate,
- de 3 % en masse à 8 % en masse et, avantageusement, de 6,6 % en masse à 7,3 % en masse d'un mélange d'acrylamide/bisacrylamide, de préférence dans un rapport massique 19/1,
- de 28 % en masse à 59 % en masse et, avantageusement, de 52,7 % en masse à 58,3 % en masse de dodécanol,
- de 22 % en masse à 53 % en masse et, avantageusement, de 22,8 % en masse à 25,2 % en masse de diméthylsulfoxyde,
- de 7 % en masse à 9 % en masse de diméthylformamide, et
- de 0,05 % en masse à 0,2 % en masse et, avantageusement, de 0,14 % en masse à 0,16 % en masse d'azobisisobutyronitrile.

Après l'étape (b) d'introduction de la solution de polymérisation dans le volume intérieur du ou des canaux du système analytique miniaturisé, on procède à l'étape (c) de polymérisation de cette solution de polymérisation pour l'obtention d'un support monolithique.

Cette étape (c) de polymérisation du procédé de préparation selon l'invention est une polymérisation par voie radicalaire en chaîne, initiée par l'amorceur de polymérisation radicalaire.

Cette étape (c) de polymérisation est avantageusement une photopolymérisation, c'est-à-dire une polymérisation réalisée par irradiation de la solution de polymérisation au moyen de rayons ultraviolets (UV). Cette irradiation peut notamment être réalisée sur une zone localisée du canal.

Dans une variante avantageuse du procédé de préparation selon l'invention, la longueur d'onde des rayons ultraviolets est comprise entre 320 nm et 380 nm, préférentiellement entre 330 nm et 370 nm et, plus préférentiellement encore, est de 347 nm, qui est le maximum d'absorption de l'AIBN.

Dans une variante avantageuse du procédé de préparation selon l'invention, la durée d'irradiation au moyen des rayons ultraviolets est comprise entre 5 min et 60 min, avantageusement entre 15 min et 45 min et, préférentiellement, est de 25 min.

À l'issue de l'étape (c), on obtient un support monolithique qui a été synthétisé *in situ* et qui est ancré sur les parois du ou des canaux du système analytique miniaturisé.

Le procédé de préparation selon l'invention comprend, après l'étape (c) de polymérisation, une étape (d) de rinçage du support monolithique. Cette étape (d) de rinçage permet d'éliminer les excès de réactifs de la solution de polymérisation n'ayant pas réagi.

Cette étape (d) de rinçage peut notamment être réalisée successivement par de l'alcool puis par de l'eau, l'alcool étant avantageusement le méthanol.

Le procédé de préparation comprend ensuite une étape (e) de mise en contact du support monolithique rincé, tel qu'obtenu à l'issue de l'étape (d), avec une solution comprenant les cations UO₂²⁺. Ce faisant, on obtient un support monolithique dans le volume intérieur du ou des canaux et sur lequel les cations UO₂²⁺ sont immobilisés.

Dans une variante avantageuse du procédé de préparation selon l'invention, la solution comprenant les ions UO₂²⁺ est préparée dans une solution aqueuse d'acétate d'ammonium.

Le suivi de la fixation des ions UO₂²⁺ sur le support monolithique peut être avantageusement effectué par couplage avec un spectromètre de masse à source plasma à couplage inductif (ICP-MS).

Dans une variante avantageuse du procédé de préparation selon l'invention, l'étape (e) de mise en contact du support monolithique avec la solution comprenant les cations UO₂²⁺ est réalisée par circulation de cette solution comprenant les cations UO₂²⁺ à travers le support monolithique.

Le procédé de préparation selon l'invention permet donc de synthétiser *in situ* un support monolithique sur lequel les cations uranyle UO₂²⁺ sont immobilisés, dans des systèmes analytiques miniaturisés dont la taille est de loin inférieure, *a minima* d'un facteur 1000, à celles des systèmes analytiques formés par des microbilles telles que celles décrites par les publications **[1]** et **[2].**

En particulier, le procédé selon l'invention permet de préparer des supports sur lesquels sont immobilisés des cations UO₂²⁺ dans des canaux dont le diamètre interne peut avantageusement être inférieur ou égal à 300 µm et, préférentiellement, peut être compris entre 50 µm et 90 µm.

La réduction d'échelle qu'il est possible d'atteindre avec le procédé de préparation selon l'invention permet aussi de réduire la consommation de solvants et de réactifs ainsi que la quantité de sous-produits de réaction et le traitement de ces derniers, ce qui présente un avantage indéniable sur le plan industriel. Cette réduction d'échelle est d'autant plus avantageuse pour des applications qui s'inscrivent dans le domaine du nucléaire, dans le sens où elle permet une réduction des contraintes liées à la manipulation d'échantillons radioactifs mais également une limitation des volumes de déchets et des coûts associés à leur gestion spécifique.

La présente invention se rapporte, en deuxième lieu, à un procédé de capture de protéines liant sélectivement l'uranium, ces protéines étant contenues dans un échantillon biologique.

Selon l'invention, ce procédé de capture comprend les étapes (i) et (ii) suivantes :
(i) la préparation, dans le volume intérieur d'au moins un canal d'un système analytique miniaturisé, d'un support monolithique sur lequel des cations UO₂²⁺ sont immobilisés, par la mise en œuvre du procédé de préparation tel que défini ci-dessus, et
(ii) au moins la circulation d'une solution contenant l'échantillon biologique à travers le support monolithique sur lequel des cations UO₂²⁺ sont immobilisés, obtenu à l'issue de l'étape (i), moyennant quoi on obtient la capture des protéines liant sélectivement l'uranium sur le support monolithique.

Dans le procédé de capture selon l'invention, lors de l'étape (i), on prépare *in situ* un support monolithique par le procédé de préparation tel que défini ci-avant, étant précisé que les caractéristiques avantageuses de ce procédé de préparation, notamment celles relatives aux modalités de mise en œuvre des étapes (a) à (e) et celles relatives au(x) canal(aux) du système analytique miniaturisé, peuvent être prises seules ou en combinaison.

Comme déjà décrit précédemment, ce support monolithique est ancré sur les parois internes du ou des canaux et comprend des ions uranyles immobilisés.

Lors de l'étape (ii) de circulation, se produit la capture sélective de la ou des protéines qui, parmi celle(s) contenue(s) dans l'échantillon biologique, présente(nt) une affinité pour les ions UO₂²⁺ qui sont immobilisés sur le support monolithique.

La capture sélective de ces protéines liant sélectivement l'uranium s'effectue donc selon le mode IMAC et ce, à partir d'un échantillon biologique dont la quantité engagée peut être drastiquement réduite par rapport à celle requise pour la capture au moyen du support des publications **[1]** et **[2].**

La présente invention se rapporte, en troisième lieu, à un procédé de récupération de protéines liant sélectivement l'uranium, ces protéines étant contenues dans un échantillon biologique.

Selon l'invention, ce procédé comprend les étapes (1) à (3) suivantes :
(1) la capture des protéines liant sélectivement l'uranium par la mise en œuvre du procédé de capture tel que défini ci-dessus,
(2) l'élimination des protéines non liées par rinçage du support monolithique par circulation d'une solution ne contenant pas de protéines, et
(3) au moins une étape d'élution, par circulation d'une solution éluante à travers le support monolithique obtenu à l'issue de l'étape (2), moyennant quoi les protéines liant sélectivement l'uranium sont récupérées dans la solution éluante.

Dans le procédé de récupération selon l'invention, lors de l'étape (1), on procède à la capture des protéines liant sélectivement l'uranium par la mise en œuvre du procédé de capture tel que défini ci-avant, étant précisé que les caractéristiques avantageuses de ce procédé de capture peuvent être prises seules ou en combinaison.

Ces protéines liant sélectivement l'uranium, qui ont été capturées lors de l'étape (1), sont ainsi récupérées du support monolithique par la mise en œuvre de l'étape (3) qui comprend au moins une étape d'élution au moyen d'une solution éluante.

Le procédé de récupération selon l'invention est de mise en œuvre particulièrement simple et permet, par élution, de récupérer, de manière sélective, les protéines liant sélectivement l'uranium qui ont été préalablement capturées sur le support monolithique sur lequel des cations UO₂²⁺ sont immobilisés.

Il est précisé que l'échantillon biologique mis en jeu dans le procédé de capture de protéines liant sélectivement l'uranium et, par conséquent, dans le procédé de récupération de protéines liant sélectivement l'uranium, peut notamment être constitué par des solutions de protéines, par des extraits cellulaires ou par des fluides biologiques, et peut notamment être issu d'une lignée cellulaire neuronale humaine.

D'autres caractéristiques et avantages de l'invention apparaîtront à la lecture du complément de description qui suit, qui se rapporte à un exemple de préparation d'un support monolithique de 8 mm sur lequel des cations uranyle sont immobilisés ainsi qu'à un exemple permettant d'illustrer les performances d'un tel support monolithique pour capturer puis récupérer des protéines liant sélectivement l'uranium contenues dans un échantillon biologique.

Bien entendu, ces exemples ne sont donnés qu'à titre d'illustration de l'objet de l'invention et ne constituent, en aucun cas, une limitation de cet objet.

### Exposé détaillé de modes de réalisation particuliers

Une solution de polymérisation de synthèse d'un support monolithique a été préparée à partir des composés suivants, dans les proportions massiques précisées ci-après :
- 2,22 mg (0,15 % en masse) d'azobisisobutyronitrile (AIBN),
- 100,05 mg (6,7 % en masse) d'un mélange acrylamide (AA) et bisacrylamide (BAA) (rapport 19/1),
- 130,60 mg (8,7 % en masse) de méthacrylate de polyéthylène glycol phosphate (EGMP),
- 346,03 mg (23,1 % en masse) de diméthylsulfoxyde (DMSO),
- 799,93 mg (53,4 % en masse) de dodécanol (DOC), et
- 118,80 mg (7,9 % en masse) de diméthylformamide (DMF).

Dans un flacon, on a successivement introduit l'AIBN, l'AA-BAA, l'EGMP, le DMSO, le DOC, puis le DMF, puis on a procédé au mélange de l'ensemble de ces composés pour obtenir la solution de polymérisation de synthèse du support monolithique.

Après dégazage, la solution de polymérisation obtenue a été introduite dans un ou plusieurs canaux des quatre canaux droits, parallèles entre eux et présentant une largeur de 50 µm, une profondeur de 50 µm et une longueur de 58,5 mm, d'un microsystème analytique en verre commercialisé par la société ChipShop.

Il est précisé, qu'avant l'introduction de cette solution de polymérisation, la surface interne du ou des canaux a été fonctionnalisée par un mélange comprenant du gamma-méthacryloxy-propyltriméthoxysilane (γ-MAPS) solubilisé dans l'acétone (50/50, v/v).

Après introduction de la solution de polymérisation dans un ou plusieurs canaux des quatre canaux, le microsystème a été placé dans un four sous un rayonnement UV d'une longueur d'onde maximale de 365 nm, à 14,5 cm de la source pendant 25 min de façon à avoir une puissance de 3,1 mW.cm⁻² afin d'obtenir la polymérisation de la solution de polymérisation.

Le support monolithique ainsi formé dans le(s) canal(aux) a été rincé au moyen d'un mélange comprenant du méthanol et de l'eau.

Une solution comprenant 500 parties par milliard (ppb) d'ions uranyle en tampon acétate d'ammonium à une concentration molaire en acétate d'ammonium comprise entre 25 mmol/L et 50 mmol/L et un pH compris entre 4 et 5, est préparée.

On fait ensuite circuler cette solution à travers le support monolithique synthétisé *in situ* et ancré sur les parois du ou des canal(aux) du microsystème, pendant 60 min, à un débit de 0,24 mL/h (4 µL/min) de manière à immobiliser les ions uranyle à la surface du support monolithique.

L'efficacité de l'immobilisation des ions uranyle est déterminée au moyen d'un spectromètre de masse à source plasma à couplage inductif (ICP-MS).

Cette étape peut être effectuée hors ligne par récupération de fractions. Mais cette étape peut avantageusement être effectuée en ligne, c'est-à-dire en continu, en couplant le microsystème avec le spectromètre de masse par l'intermédiaire d'un micro-nébuliseur. Dans le cas du présent exemple, 50 ng d'uranium ont été fixés sur le support monolithique de 8 mm.

Dans le cas où le spectromètre de masse est un ICP triple quadripôle, le signal du phosphore et/ou du soufre contenus dans les protéines peut également être suivi en plus de celui de l'uranium, lors du suivi de la capture des protéines.

La possibilité de suivre aussi bien les ions uranyles que leurs molécules-cibles est un avantage considérable, ceci permettant de déterminer à la fois l'efficacité d'immobilisation de ces ions sur le support monolithique et sa capacité à capturer des molécules-cibles, par interaction avec les ions immobilisés. À titre d'exemple, la quantité de la protéine apotransferrine capturée par ce support monolithique sur lequel les ions uranyle sont immobilisés et récupérée, est de 0,75 µg.

Le tableau 1 ci-dessous fait état de la quantité d'uranium immobilisé sur le support monolithique préparé par le procédé selon l'invention, ainsi que de la quantité d'apotransferrine récupérée suite à sa capture par ce même support, par comparaison aux quantités obtenues avec le support de référence formé par les microbilles tel que décrit dans la publication [1].

**Tableau 1**

| | Support microbilles (de référence) | Support monolithique (selon l'invention) |
|---|---|---|
| Volume du support | 50 µL d'une solution de 1,1 g de microbilles/mL | 16 nL (8 mm x 50 µm x 50 µm) |
| Quantité d'uranium immobilisé | 5,95 mg | 50 ng |
| Quantité d'apotransferrine éluée | 40 µg | 0,75 µg |

### Références

**[1]** C. Basset et al., Journal of Chromatography A, 2008, 1185, pages 233-240
**[2]** A. Dedieu et al., Journal of Chromatography A, 2009, 1216, pages 5365-5376
**[3]** M. Araya-Farias et al., Analyst, 2017, 142, pages 485-494

## Revendications

1. Procédé de préparation, dans le volume intérieur d'au moins un canal d'un système analytique miniaturisé, d'un support monolithique sur lequel des cations UO₂²⁺ sont immobilisés, ce procédé comprenant les étapes (a) à (e) successives suivantes :
(a) l'activation de la surface interne du ou des canaux ;
(b) l'introduction d'une solution de polymérisation de synthèse d'un support monolithique dans le volume intérieur du ou des canaux, la solution de polymérisation comprenant :
- un monomère comprenant un groupement phosphate,
- au moins un agent de réticulation,
- plusieurs solvants, et
- un amorceur de polymérisation radicalaire ;
(c) la polymérisation de la solution de polymérisation obtenue à l'étape (b), moyennant quoi on obtient un support monolithique ancré sur les parois du ou des canaux ;
(d) le rinçage du support monolithique obtenu à l'étape (c) ; et
(e) la mise en contact du support monolithique rincé obtenu à l'étape (d) avec une solution comprenant des cations UO₂²⁺, moyennant quoi on obtient un support monolithique sur lequel les cations UO₂²⁺ sont immobilisés.

2. Procédé selon la revendication 1, dans lequel le monomère comprenant un groupement phosphate est un monomère méthacrylate, de préférence le méthacrylate de polyéthylène glycol phosphate.

3. Procédé selon la revendication 1 ou 2, dans lequel l'agent de réticulation est composé d'un mélange acrylamide/bisacrylamide.

4. Procédé selon l'une quelconque des revendications 1 à 3, dans lequel les solvants sont choisis parmi le dodécanol, le diméthylformamide et le diméthylsulfoxyde.

5. Procédé selon l'une quelconque des revendications 1 à 4, dans lequel l'amorceur de polymérisation radicalaire est l'azobisisobutyronitrile (AIBN).

6. Procédé selon la revendication 5, dans lequel la solution de polymérisation comprend, par rapport à la masse totale de la solution de polymérisation :
- de 0,05 % en masse à 0,2 % en masse et, avantageusement, de 0,14 % en masse à 0,16 % en masse d'azobisisobutyronitrile,
- de 3% en masse à 8 % en masse et, avantageusement, de 6,6 % en masse à 7,3 % en masse d'acrylamide et de bisacrylamide,
- de 4% en masse à 10 % en masse et, avantageusement, de 8,7 % en masse à 9,7 % en masse de méthacrylate de polyéthylène glycol phosphate,
- de 22 % en masse à 53 % en masse et, avantageusement, de 22,8 % en masse à 25,2 % en masse de diméthylsulfoxyde,
- de 28% en masse à 59 % en masse et, avantageusement, de 52,7 % en masse à 58,3 % en masse de dodécanol, et
- de 7% en masse à 9 % en masse de diméthylformamide.

7. Procédé selon l'une quelconque des revendications 1 à 6, dans lequel l'étape (c) de polymérisation est réalisée par irradiation au moyen de rayons ultraviolets, la longueur d'onde de ces rayons ultraviolets étant avantageusement comprise entre 320 nm et 380 nm, préférentiellement entre 330 nm et 370 nm et, plus préférentiellement encore, étant de 347 nm.

8. Procédé selon la revendication 7, dans lequel, dans l'étape (c), la durée d'irradiation au moyen des rayons ultraviolets est comprise entre 5 min et 60 min, avantageusement entre 15 min et 45 min et, préférentiellement, est de 25 min.

9. Procédé selon l'une quelconque des revendications 1 à 8, dans lequel l'étape (d) de rinçage est réalisée successivement avec un alcool puis avec de l'eau, l'alcool étant avantageusement le méthanol.

10. Procédé selon l'une quelconque des revendications 1 à 9, dans lequel, le ou les canaux étant en verre, l'étape (a) d'activation est réalisée par silanisation, avantageusement au moyen de gamma-méthacryloxypropyltriméthoxysilane (γ-MAPS) comme agent de silanisation.

11. Procédé selon l'une quelconque des revendications 1 à 10, dans lequel le diamètre interne du ou des canaux est avantageusement inférieur ou égal à 300 µm et, préférentiellement, est compris entre 50 µm et 90 µm.

12. Procédé selon l'une quelconque des revendications 1 à 11, dans lequel la solution comprenant les cations UO₂²⁺ est préparée dans une solution aqueuse d'acétate d'ammonium.

13. Procédé selon l'une quelconque des revendications 1 à 12, dans lequel l'étape (e) de mise en contact du support monolithique avec la solution comprenant les cations UO₂²⁺ est réalisée par circulation de cette solution comprenant les cations UO₂²⁺ sur le support monolithique.

14. Procédé de capture de protéines liant sélectivement l'uranium, ces protéines étant contenues dans un échantillon biologique, ce procédé comprenant les étapes (i) et (ii) suivantes :
(i) la préparation, dans le volume intérieur d'au moins un canal d'un système analytique miniaturisé, d'un support monolithique sur lequel des cations UO₂²⁺ sont immobilisés, par la mise en œuvre du procédé de préparation selon l'une quelconque des revendications 1 à 13, et
(ii) au moins la circulation d'une solution contenant l'échantillon biologique sur le support monolithique sur lequel des cations UO₂²⁺ sont immobilisés, obtenu à l'issue de l'étape (i), moyennant quoi on obtient la capture des protéines liant sélectivement l'uranium sur le support monolithique.

15. Procédé de récupération de protéines liant sélectivement l'uranium, ces protéines étant contenues dans un échantillon biologique, ce procédé comprenant les étapes (1) à (3) suivantes :
(1) la capture des protéines liant sélectivement l'uranium par la mise en œuvre du procédé de capture selon la revendication 14,
(2) l'élimination des protéines non liées par rinçage du support monolithique par circulation d'une solution ne contenant pas de protéines, et
(3) au moins une étape d'élution, par circulation d'une solution éluante à travers le support monolithique obtenu à l'issue de l'étape (2), moyennant quoi les protéines liant sélectivement l'uranium sont récupérées dans la solution éluante.

## Patentansprüche

1. Verfahren zur Herstellung eines monolithischen Trägers im Innenraum mindestens eines Kanals eines miniaturisierten Analysesystems, auf dem UO₂²⁺-Kationen immobilisiert sind, wobei dieses Verfahren die folgenden aufeinanderfolgenden Schritte (a) bis (e) umfasst:
(a) Aktivieren der inneren Oberfläche des Kanals/der Kanäle;
(b) Einbringen einer Polymerisationslösung zur Synthese eines monolithischen Trägers in das Innenvolumen des/der Kanals/Kanäle, wobei die Polymerisationslösung Folgendes umfasst:
- ein Monomer, das eine Phosphatgruppe umfasst,
- mindestens ein Vernetzungsmittel,
- mehrere Lösungsmittel und
- einen Radikalpolymerisationsinitiator;
(c) Polymerisieren der in Schritt (b) erhaltenen Polymerisationslösung, wodurch ein monolithischer Träger erhalten wird, der an den Wänden des oder der Kanäle verankert ist;
(d) Spülen des in Schritt (c) erhaltenen monolithischen Trägers; und
(e) Inkontaktbringen des in Schritt (d) erhaltenen gespülten monolithischen Trägers mit einer Lösung, die UO₂²⁺⁻Kationen umfasst, wodurch ein monolithischer Träger erhalten wird, auf dem die UO₂²⁺⁻Kationen immobilisiert sind.

2. Verfahren nach Anspruch 1, wobei das Monomer, das eine Phosphatgruppe umfasst, ein Methacrylatmonomer ist, vorzugsweise Polyethylenglykolphosphatmethacrylat.

3. Verfahren nach Anspruch 1 oder 2, wobei das Vernetzungsmittel aus einem Gemisch aus Acrylamid/Bisacrylamid besteht.

4. Verfahren nach einem der Ansprüche 1 bis 3, wobei die Lösungsmittel aus Dodecanol, Dimethylformamid und Dimethylsulfoxid ausgewählt sind.

5. Verfahren nach einem der Ansprüche 1 bis 4, wobei der Radikalpolymerisationsinitiator Azobisisobutyronitril (AIBN) ist.

6. Verfahren nach Anspruch 5, wobei die Polymerisationslösung bezogen auf die Gesamtmasse der Polymerisationslösung Folgendes umfasst:
- 0,05 Massenprozent bis 0,2 Massenprozent und vorteilhafterweise 0,14 Massenprozent bis 0,16 Massenprozent Azobisisobutyronitril,
- 3 Massenprozent bis 8 Massenprozent und vorteilhafterweise 6,6 Massenprozent bis 7,3 Massenprozent Acrylamid und Bisacrylamid,
- 4 Massenprozent bis 10 Massenprozent und vorteilhafterweise 8,7 Massenprozent bis 9,7 Massenprozent Polyethylenglykolphosphatmethacrylat,
- 22 Massenprozent bis 53 Massenprozent und vorteilhafterweise 22,8 Massenprozent bis 25,2 Massenprozent Dimethylsulfoxid,
- 28 Massenprozent bis 59 Massenprozent und vorteilhafterweise 52,7 Massenprozent bis 58,3 Massenprozent Dodecanol, und
- 7 Massenprozent bis 9 Massenprozent Dimethylformamid.

7. Verfahren nach einem der Ansprüche 1 bis 6, wobei der Schritt des Polymerisierens (c) durch Bestrahlen mit ultravioletten Strahlen durchgeführt wird, wobei die Wellenlänge dieser ultravioletten Strahlen vorteilhafterweise zwischen 320 nm und 380 nm, vorzugsweise zwischen 330 nm und 370 nm und noch bevorzugter 347 nm beträgt.

8. Verfahren nach Anspruch 7, wobei in Schritt (c) die Bestrahlungsdauer mit ultravioletten Strahlen zwischen 5 min und 60 min, vorteilhafterweise zwischen 15 min und 45 min und vorzugsweise 25 min beträgt.

9. Verfahren nach einem der Ansprüche 1 bis 8, wobei der Schritt (d) des Spülens nacheinander mit Alkohol und dann mit Wasser durchgeführt wird, wobei der Alkohol vorteilhafterweise Methanol ist.

10. Verfahren nach einem der Ansprüche 1 bis 9, wobei der oder die Kanäle aus Glas bestehen und der Schritt des Aktivierens (a) durch Silanisieren durchgeführt wird, vorteilhafterweise unter Verwendung von Gamma-Methacryloxypropyltrimethoxysilan (γ-MAPS) als Silanisierungsmittel.

11. Verfahren nach einem der Ansprüche 1 bis 10, wobei der Innendurchmesser des oder der Kanäle vorteilhafterweise kleiner oder gleich 300 µm ist und vorzugsweise zwischen 50 µm und 90 µm liegt.

12. Verfahren nach einem der Ansprüche 1 bis 11, wobei die Lösung, die die UO₂²⁺⁻Kationen umfasst, in einer wässrigen Ammoniumacetatlösung hergestellt wird.

13. Verfahren nach einem der Ansprüche 1 bis 12, wobei der Schritt (e) des Kontaktierens des monolithischen Trägers mit der Lösung, die die UO₂²⁺-Kationen umfasst, durch Zirkulieren dieser Lösung, die die UO₂²⁺⁻Kationen umfasst, durch den monolithischen Träger durchgeführt wird.

14. Verfahren zum Einfangen von Proteinen, die Uran selektiv binden, wobei diese Proteine in einer biologischen Probe enthalten sind, wobei dieses Verfahren die folgenden Schritte (i) und (ii) umfasst:
(i) Herstellen eines monolithischen Trägers, auf dem UO₂²⁺-Kationen immobilisiert sind, im Innenraum mindestens eines Kanals eines miniaturisierten Analysesystems durch Anwendung des Herstellungsverfahrens nach einem der Ansprüche 1 bis 13, und
(ii) mindestens das Zirkulieren einer die biologische Probe enthaltenden Lösung durch den monolithischen Träger, auf dem UO₂²⁺⁻Kationen immobilisiert sind, der am Ende des Schritts (i) erhalten wird, wodurch das selektive Einfangen der Proteine auf dem monolithischen Träger erreicht wird.

15. Verfahren zum Gewinnen von Proteinen, die Uran selektiv binden, wobei diese Proteine in einer biologischen Probe enthalten sind, wobei dieses Verfahren die folgenden Schritte (1) bis (3) umfasst:
(1) das Einfangen von Uran-bindenden Proteinen durch das Durchführen des Einfangverfahrens nach Anspruch 14,
(2) Entfernen ungebundener Proteine durch Spülen des monolithischen Trägers durch Zirkulieren einer proteinfreien Lösung, und
(3) mindestens einen Schritt des Eluierens durch Zirkulieren einer Elutionslösung durch den nach Schritt (2) erhaltenen monolithischen Träger, wodurch die selektiv an Uran bindenden Proteine in der Elutionslösung zurückgewonnen werden.

## Claims

1. A method for preparing, in the internal volume of at least one channel of a miniaturised analytical system, a monolithic support on which UO₂²⁺ cations are immobilised, which method comprises the following successive steps (a) to (e) of:
(a) activating the inner surface of the channel(s);
(b) introducing a polymerisation solution for synthesising a monolithic support into the internal volume of the channel(s), the polymerisation solution comprising:
- a monomer comprising a phosphate group,
- at least one crosslinking agent
- several solvents, and
- a radical polymerisation initiator;
(c) polymerising the polymerisation solution obtained in step (b), whereby a monolithic support anchored onto the walls of the channel(s) is obtained;
(d) rinsing the monolithic support obtained in step (c); and
(e) contacting the rinsed monolithic support obtained in step (d) with a solution comprising UO₂²⁺ cations, whereby a monolithic support on which the UO₂²⁺ cations are immobilized is obtained.

2. The method according to claim 1, wherein the monomer comprising a phosphate group is a methacrylate monomer, preferentially polyethylene glycol methacrylate phosphate.

3. The method according to claim 1 or 2, wherein the crosslinking agent is composed of a mixture of acrylamide/bisacrylamide.

4. The method according to any of claims 1 to 3, wherein the solvents are selected from dodecanol, dimethylformamide and dimethylsulphoxide.

5. The method according to any of claims 1 to 4, wherein the radical polymerisation initiator is azobisisobutyronitrile (AIBN).

6. The method according to claim 5, wherein the polymerisation solution comprises, based on the total mass of the polymerisation solution:
- from 0.05 mass % to 0.2 mass % and, advantageously, from 0.14 mass % to 0.16 mass % of azobisisobutyronitrile,
- from 3 mass % to 8 mass % and, advantageously, from 6.6 mass % to 7.3 mass % of acrylamide and bisacrylamide,
- from 4 mass % to 10 mass % and, advantageously, from 8.7 mass % to 9.7 mass % of polyethylene glycol methacrylate phosphate,
- from 22 mass % to 53 mass % and, advantageously, from 22.8 mass % to 25.2 mass % of dimethylsulphoxide,
- from 28 mass % to 59 mass % and, advantageously, from 52.7 mass % to 58.3 mass % of dodecanol, and
- from 7 mass % to 9 mass % dimethylformamide.

7. The method according to any of claims 1 to 6, wherein the polymerisation step (c) is carried out by irradiation by means of ultraviolet rays, the wavelength of these ultraviolet rays being advantageously between 320 nm and 380 nm, preferentially between 330 nm and 370 nm and, even more preferentially, being 347 nm.

8. The method according to claim 7, wherein, in step (c), the duration of irradiation by means of the ultraviolet rays is between 5 min and 60 min, advantageously between 15 min and 45 min and, more preferentially, is 25 min.

9. The method according to any of claims 1 to 8, wherein the rinsing step (d) is carried out successively with an alcohol and then with water, the alcohol being advantageously methanol.

10. The method according to any of claims 1 to 9, wherein, the channel(s) being made of glass, the activation step (a) is carried out by silanisation, advantageously by means of gamma-methacryloxypropyltrimethoxysilane (γ-MAPS) as silanising agent.

11. The method according to any of claims 1 to 10, wherein the internal diameter of the channel(s) is advantageously less than or equal to 300 µm and, preferentially, is between 50 µm and 90 µm.

12. The method according to any of claims 1 to 11, wherein the solution comprising the UO₂²⁺ cations is prepared in an aqueous solution of ammonium acetate.

13. The method according to any of claims 1 to 12, wherein step (e) of contacting the monolithic support with the solution comprising the UO₂²⁺ cations is carried out by circulating this solution comprising the UO₂²⁺ cations through the monolithic support.

14. A method for capturing proteins that selectively bind uranium, these proteins being contained in a biological sample, this method comprising the following steps (i) and (ii) of:
(i) preparing, in the internal volume of at least one channel of a miniaturised analytical system, a monolithic support on which UO₂²⁺ cations are immobilised, by implementing the preparation method according to any of claims 1 to 13, and
(ii) at least circulating a solution containing the biological sample through the monolithic support on which UO₂²⁺ cations are immobilised, obtained at the end of step (i), whereby the capture of the proteins that selectively bind uranium on the monolithic support is achieved.

15. A method for recovering proteins that selectively bind uranium, these proteins being contained in a biological sample, this method comprising the following steps (1) to (3) of:
(1) capturing the proteins that selectively bind uranium by implementing the capture method of claim 14,
(2) removing the unbound proteins by rinsing the monolithic support by circulating a solution containing no proteins, and
(3) at least one elution step, by circulating an eluting solution through the monolithic support obtained at the end of step (2), whereby the proteins that selectively bind uranium are recovered in the eluting solution.
